# EUROPEAN PATENT SPECIFICATION

(11) **EP 3 212 069 B1**
(45) Date of publication and mention of the grant of the patent: **09.09.2020**
(21) Application number: 15855427.9
(22) Date of filing: 30.10.2015
(51) Int. Cl.: G02B 6/38

(54) **MEMBRANE-FREE FIBER BRAGG GRATING PRESSURE SENSING GUIDEWIRE**
DRUCKMESSUNGSFÜHRUNGSDRAHT AUS MEMBRANFREIEM FASER-BRAGG-GITTER
GUIDE POUR CAPTEUR DE PRESSION À RÉSEAUX DE BRAGG SUR FIBRE SANS MEMBRANE

(30) Priority: 31.10.2014 US 201462073203 P
(43) Date of publication of application: 06.09.2017
(73) Proprietor: Lake Region Medical, Inc., Chaska, MN 55318 (US)
(72) Inventor: HAYES, John, Michael, Cork (IE); KAT, Pieter, Lucas, 1724 NK Oudkarspel (NL)
(74) Representative: Mathys & Squire
(86) International application number: PCT/US2015/058408
(87) International publication number: WO 2016/070099

(56) References cited:
- JP-A- S60 260 010
- US-A- 4 201 445
- US-A1- 2006 011 820
- US-A1- 2008 281 209
- US-A1- 2012 197 097
- US-A1- 2013 022 308
- US-A1- 2013 317 372
- US-A1- 2014 180 030

## Description

### Field of the Invention

This disclosure relates to the field of medical devices, and specifically to catheter systems including guidewires for use in the collection of diagnostic information, such as for example pressure, from multiple sites within a body lumen.

### Summary

A known technique of comparing pressures on either side of an affected area of a vessel to determine if additional treatment is necessary is known as Fractional Flow Reserve or FFR. Examples of devices and methods used in FFR procedures are shown and described in U.S. Patent 5,987,995 and U.S. Patent 6,868,736.

Embodiments of the present disclosure include catheter systems, and particularly those that include a diagnostic guidewire assembly equipped with one or more pressure sensors that can be placed across a lesion, occlusion or other affected area within a vessel and then near-simultaneously detect pressures on either side of the affected area.

The diagnostic guidewire assembly and system of the present disclosure provides a benefit over known FFR systems in that upstream and downstream pressures are detected simultaneously, with a distal region of the guidewire assembly positioned across the affected region. With the guidewire assembly in place, the detected pressures are analyzed, and depending on their values, a determination of whether further treatment of the affected area is required occurs without removal of the guidewire. If it is determined that further treatment (such as balloon angioplasty, stent delivery, etc.) is required, the diagnostic guidewire assembly remains in place to guide the subsequent treatment system (POBA catheter, stent delivery catheter, etc.) to the affected area. In addition, following the therapeutic treatment, the guidewire assembly can remain in place to conduct a follow-up simultaneous FFR diagnosis procedure to determine the efficacy of the therapeutic treatment. This process may be repeated as needed, with the guidewire assembly remaining in place throughout the one or more diagnostic and therapeutic procedures.

The ability to conduct such improved simultaneous FFR diagnosis with the same guidewire that can be used to advance the treatment catheter to the affected site of the vessel is not only more efficient than multiple-wire systems, it also minimizes irritation to the vessel and reduces the risk of embolization.

Stenting and angioplasty devices and procedures are well known and understood by those of skill in the art. A description of such procedures and example devices may be found in U.S. Patent 4,886,062.

Embodiments of the aforementioned diagnostic guidewire system can utilize a variety of sensors and sensory techniques to detect pressure values. The guidewire is equipped with a fiber optic wire. At a distal region of the fiber optic wire are a plurality of pressure sensors. Each pressure sensor is comprised of at least one fiber Bragg grating (FBG), with each FBG having a distinct grating period to provide correspondingly distinct peak reflection wavelengths of reflected light through the fiber optic wire. Precise monitoring of the spectral peak position of the light returned from each FBG is analyzed and compared, via an interrogator (light receiver), to provide a pressure difference between the upstream and downstream values. It may be preferable to select FBG characteristics such that the reflected signals from the FBGs do not overlap in wavelength output.

The portion of the fiber containing a pressure-sensing FBG is exposed directly to blood. Pressure is exerted on the fiber optic wire by the contact with the blood. The fiber optic cable can be in contact with the guidewire. The exerted pressure does not laterally move the fiber optic cable, but instead compresses the fiber optic cable. The pressure on the fiber optic cable impacts the refractive index of the core, thereby shifting the wavelength of light that is reflected by the FBG. A second temperature-sensing FBG can be positioned on the fiber optic cable proximal to the pressure-sensing FBG. The temperature-sensing FBG is not in contact with blood, and does not experience any compressive forces due to the pressure of the blood. Due to its proximity to the pressure-sensing FGB, the temperature-sending FBG will be impacted in a nearly identical fashion by the local temperature within the blood vessel as the pressure-sensing FBG. In this manner, any shifts in reflected wavelength in the pressure-sensing FBG due to temperature will be identified by the same shift in reflected wavelength in the temperature-sensing FBG, thereby allowing the detection of the shift in reflected wavelength that is due solely to pressure. This shift in reflected wavelengths can then be used to determine the pressure that the blood applies directly to the portion of the fiber optic wire containing the pressure-sensing FBG.

In another embodiment, the portion of the fiber optic wire containing the pressure-sensing FBG is constrained to prevent any strain on the fiber optic wire at this location. The physical constraint can take the form of a cage that can be embedded within an interior of the guidewire.

Examples of a systems using FBGs and an interrogator system for analyzing reflected light is described in U.S. Publication 2014/0363126, to P.L. Kat and filed June 5, 2014, and U.S. Patent 8,345,238.

These and other embodiments of the invention are disclosed herein and are illustrated in for following drawings.

US2013/0317372 discloses an optical fiber pressure sensor guidewire.

Aspects of the invention are recited in the independent claims and preferred features are recited in the dependent claims.

### Brief Description of the Drawings

FIG. 1 is a schematic view of a guidewire system in its environment of use.
FIG. 2 is a schematic isolated view of the guidewire system shown in FIG. 1.
FIG. 3a is a perspective view of an embodiment of a distal region of the guidewire assembly shown in FIG. 2.
FIG. 3b is a cross-sectional view of the guidewire assembly shown in FIG. 3a.
FIG. 3c is a perspective view of the guidewire assembly shown in FIG. 3a with a coating applied.
FIG. 4a is a perspective view of an embodiment of a distal end region of the guidewire assembly shown in FIG. 2.
FIG. 4b is a cross-sectional view of the guidewire assembly shown in FIG. 4a.
FIG. 4c is a perspective view of the guidewire assembly shown in FIG. 4a with a coating applied.
FIG. 5a is a perspective view of an embodiment of a distal end region of the guidewire assembly shown in FIG. 3.
FIG. 5b is a cross-sectional view of the guidewire assembly shown in FIG. 5a.
FIG. 6a is a perspective view of an embodiment of a distal end region of the guidewire assembly shown in FIG. 3.
FIG. 6b is a cross-sectional view of the guidewire assembly shown in FIG. 6a.
FIG. 6c is a perspective view of the distal fiber optic wire assembly shown in FIG. 6a
FIG. 7 is a detailed view of the distal end region of the guidewire system shown in FIG. 1 positioned across a lesion site within a body vessel.
FIG. 8 is a schematic view showing elements of the system and the wavelength shift of light interaction with FBGs.
FIG. 9 is a cross-sectional view of a connector for connecting proximal and distal optic fibers.

### Detailed Description

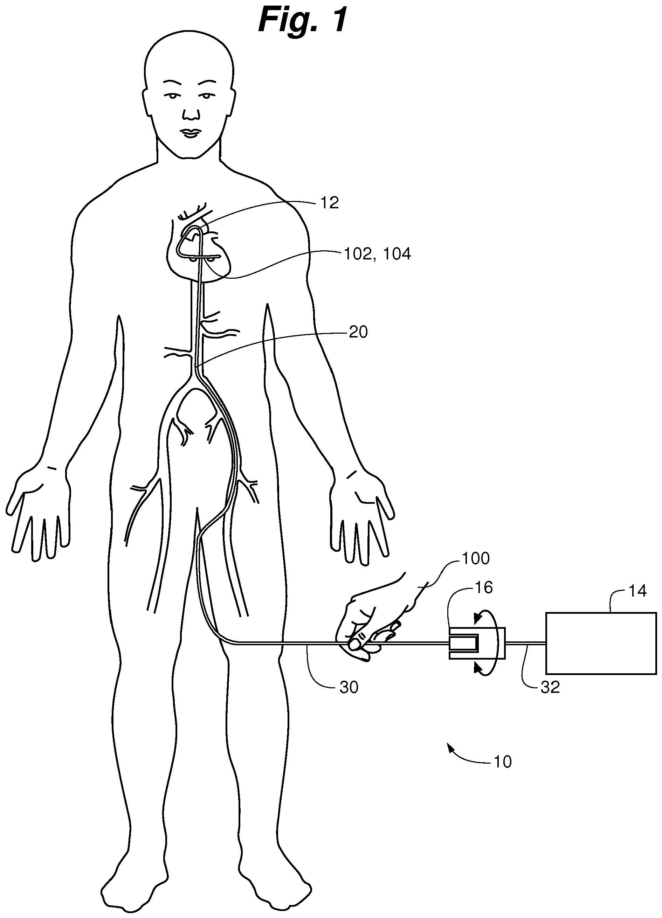

In the embodiments described herein, and shown in the the various FIGs. 1-13, the system 10 can be viewed as being comprised of three primary elements or assemblies in the following manner:

As seen in FIG. 1, a distal or guidewire assembly 12 of the system 10 is comprised primarily of a guidewire 20 and a distal fiber optic wire 30, as well as associated elements described in greater detail below.

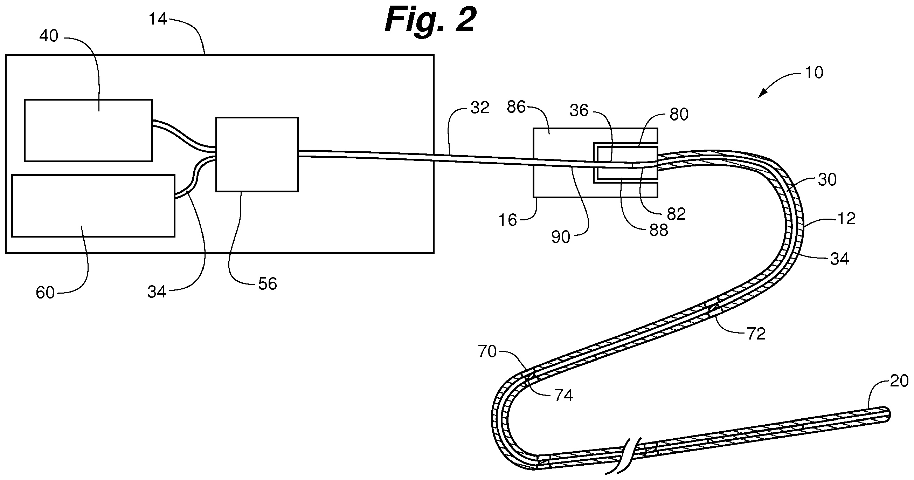

As seen in FIG. 2 a proximal assembly 14 of the system 10 is comprised of a proximal fiber optic wire or pathway of fibers 32, a light source 40, a circulator 56, a light detection element (or light wavelength detector) 60 as well as other components for sending and analyzing light signal(s) transmitted through the fiber optic wires 30 and 32.

Connecting the distal guidewire assembly 12 and the proximal assembly 14 is a connection assembly or connector 16 which connects the distal fiber optic wire 30 and proximal fiber optic wire 32. Embodiments of the system 10 shown with assemblies 12, 14 connected by connector 16 are depicted in FIGs. 1, 2 and 9.

In FIG. 1 a schematic view is provided, which illustrates an example use of the system 10 in a diagnostic FFR procedure. In such a procedure the guidewire assembly 12, via Seldinger or other technique, is introduced into the vasculature. Under fluoroscopy or other imaging techniques, the guidewire assembly 12 is advanced by the physician or practitioner (represented by the depicted hand) 100 to an affected region 102 of a vessel 104, such as is shown in FIG. 2. The affected region 102 may be a lesion, occlusion or other abnormality within the vessel 104 causing restriction in blood flow there through.

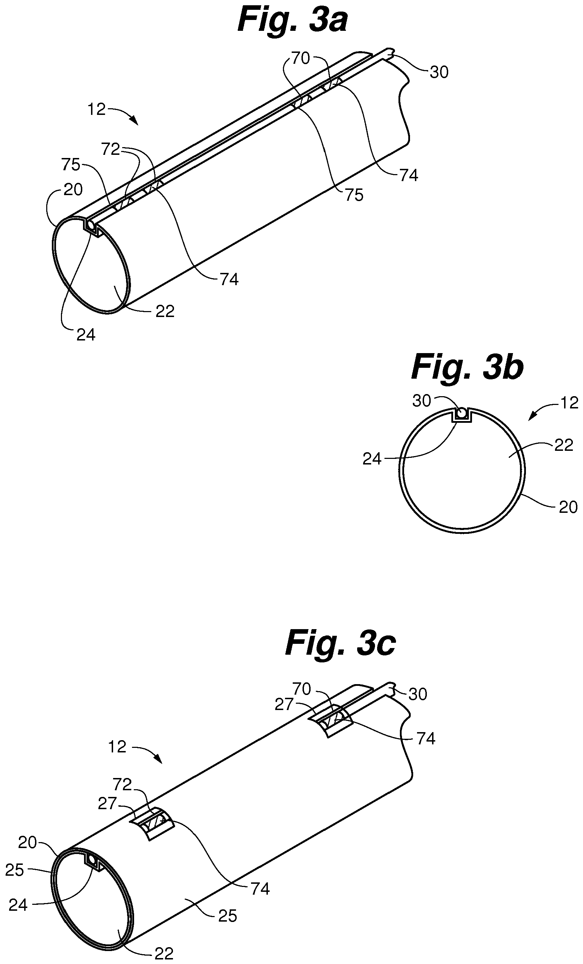

The guidewire assembly 12 may have a variety of configurations, some examples of which are illustrated in FIGs. 3a-6c. Common to all configurations is the presence of a guidewire 20 having a guidewire body or shaft 22, which supports the distal fiber optic wire 30.

The distal fiber optic wire 30 includes one or more sensor locations (or "sensor stations"), such as stations 70 and 72 shown. Each sensor station 70, 72 is comprised of at least one pressure-sensing Fiber Bragg Grating (FBG) 74 within the distal fiber optic wire 30. In one embodiment, each sensor station 70, 72 further comprises a temperature-sensing FBG 75 that is not responsive to pressure. Note that while the temperature-sensing FBG 75 is not responsive to pressure, the pressure-sensing FBG 74 is responsive to temperature. In fact, the primary function of the temperature-sensing FBG 75 is to compensate for the impact of temperature on the pressure-sensing FBG 74. While the use and function of the FBGs 74 and 75 within the sensor stations 70,72 are discussed in greater detail below, it should be noted that in the various embodiments shown and described herein a key feature of the present invention is to configure the guidewire assembly 12 in such a manner that at least those regions of the fiber optic wire 30 which include pressure-sensing FBGs 74 are directly exposed to the vascular environment. That is to say: the region or regions of the fiber optic wire 30 which include a pressure-sensing FBG 74 is directly exposed to blood within the interior of the vessel without any additional membranes (the optical wire 30 and/or the guidewire 20 are membrane-free), sleeves or other structures interposed between the sensor station and the vessel environment. In this manner environmental conditions of the vessel (such as blood pressure) directly affects the pressure-sensing FBG 74 without interference or enhancement by intervening structure.

The examples of the guidewire assembly 12 shown in FIGs. 3a-6c illustrate some types of possible configurations that provide the preferred FBG exposure.

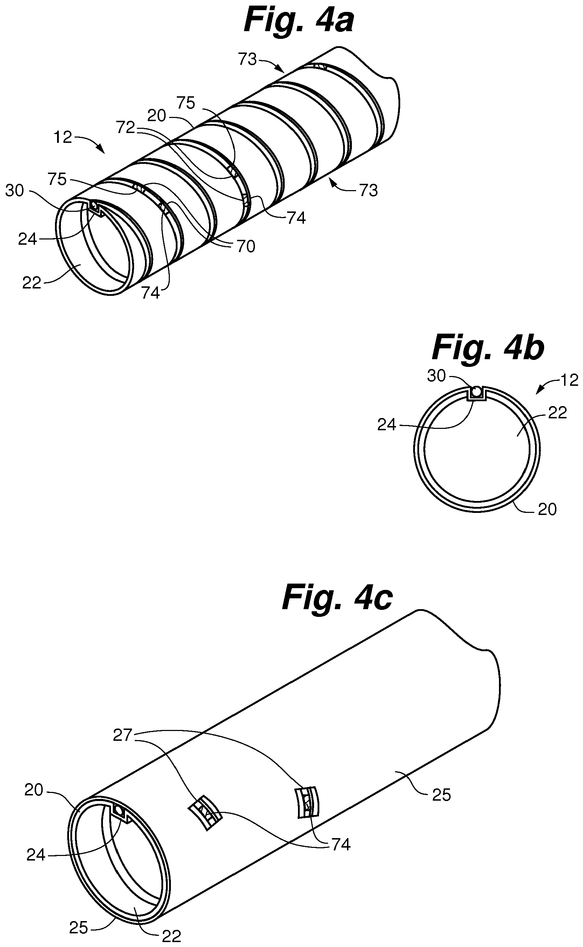

In FIGs 3a, 3b, and 3c, the guidewire shaft 22 defines a linear groove or channel 24. The distal fiber optic wire 30 is positioned at least partially within and extends along the channel 24. This configuration provides protection to the fiber optic wire 30 yet still allows the FBGs 74, 75 of the sensor stations 70 and 72 to remain exposed through the channel opening 26. In this configuration, the upper surface of the distal fiber optic wire 30 at the locations of the FBGs 74, 75 are exposed on their upper surface, while the lower surface at these locations abuts the channel 24. In the preferred embodiment, the portions of the distal fiber optic wire 30 containing the pressure-sensing FBGs 74 are exposed directly to blood in the vessel environment in order for the blood pressure to directly impact the pressure-sensing FBGs 74. At the same time, portions containing the temperature-sensing FBGs 75 are shielded from direct contact with the blood as to avoid having blood pressure alter the signal received at the temperature-sensing FBGs 75. As shown in Figure 4c, this is accomplished by covering guidewire shaft 22 and the distal fiber optic wire 30 with a coating surface 25. Windows 27 in the coating 25 expose those portions of the distal fiber optic wire 30 that contain the pressure-sensing FBGs 74. This coating 25 should be sufficiently pressure isolating so as to prevent pressure on the outside of the coating 25 from impacting the temperature-sensing FBGs 75 under the coating 25. At the same time, the coating 25 is a sufficiently good thermal conductor that the temperature of each temperature-sensing FBG 75 is approximately equal to the temperature of the pressure-sensing FBG 74. As explained in more detail below, this allows the signal received from light reflecting from the temperature-sensing FBG 75 to be used to eliminate the impact of temperature on the signal received from light reflecting from the pressure-sensing FBG 74.

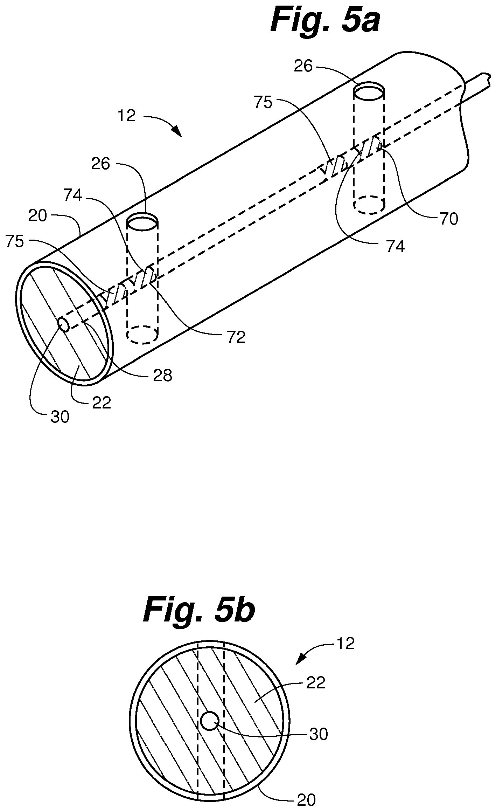

In FIGs. 4a, 4b, and 4c, an alternative configuration is shown wherein the channel 24 is a helical grove. The helical grove channel 24 provides similar protection to the fiber optic wire 30 and also allows the sensors 70 and 72 to be displaced around the circumference of the guidewire 20. This allows the sensor stations 70, 72, etc. to provide a 360-degree sensory window of the vessel lumen. As was the case in FIGs 4a-4c, each sensor station 70, 72 preferably comprises a pressure-sensing FBG 74 and a temperature-sensing FBG 75. A coating(s), layer(s) or sleeve (s) of material 25 is applied over the top of the guidewire shaft 22 and the distal fiber optic wire 30, with windows 27 in the coating 25 exposing the pressure-sensing FBG 74. In Figure 5a and 5c, only two sensor stations 70, 72 are shown, with two additional sensor stations 73 being positioned on the opposite side of the guidewire shaft 22.

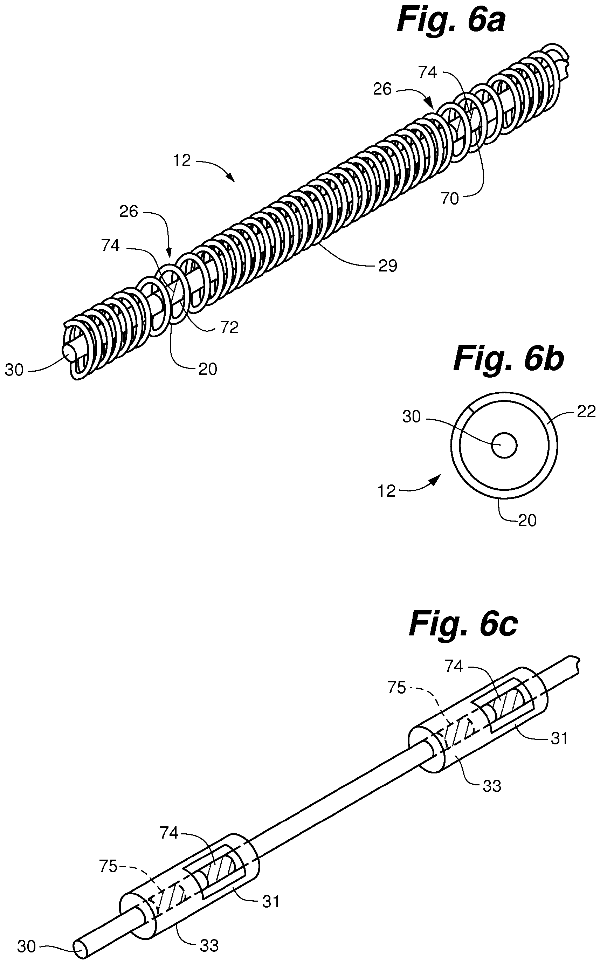

In FIGs. 5a and 5b, the guidewire 20 is configured as a hypotube or catheter shaft with lumen 28 extending there through. The distal fiber optic wire 30 is contained within the lumen 28. The guidewire shaft 22 will include openings 26 which provide a sensory window corresponding to the position of the pressure-sensing FBG 74 of each sensor station 70 and 72. In the embodiment shown in FIGs 6a and 6b, an upper and lower opening are provided at each pressure-sensing FBG 74. This exposes both the top and the bottom surface of the pressure-sensing FBG 74 to the interior of the vessel. In other embodiments, only a single opening to each pressure-sensing FBG 74 is provided through the hypotube. In still further embodiments, three or more openings are provided to expose each pressure-sensing FBG 74 to three or more locations in the vessel. In these embodiments, it is anticipated that each opening 26 will extend radially outward from the distal fiber optic wire 30, with the fiber optic wire 30 residing approximately at the center of the hypotube. However, other orientations of the openings 26 and the fiber optic wire 30 within the hypotube are within the scope of the present disclosure. In FIGs. 5a, the temperature-sensing FBGs 75 are not exposed to the openings 62, and therefore will not be sensitive to the pressure of blood that enters through those openings 62. Nonetheless, the temperature-sensing FBGs 75 are positioned proximal to the openings and the pressure-sensing FBGs 74 so that the temperature of the blood at the opening 62 will impact on and be registered by the temperature sensing FBG 75.

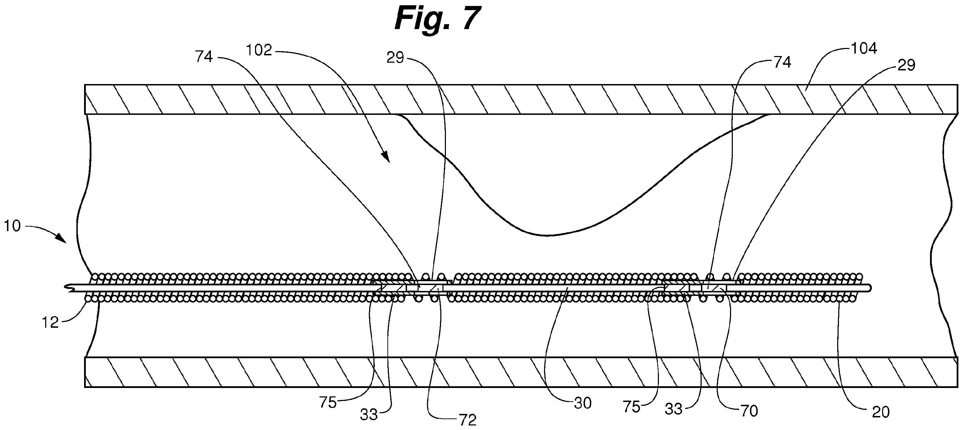

In FIGs. 6a, 6b, and 6c, an embodiment of the guidewire assembly 12 is shown in accordance with the invention wherein the core 22 of the guidewire 20 is space wound in a single filar coil or multifilar coils 29 which forms a lumen 28 through which the distal fiber optic wire 30 extends. As shown best in FIG 6a, the tightness or frequency of the the coils 29 are configured such that the coils 29 form openings 26 which allow the pressure-sensing FBGs 74 at sensor stations 70 and 72 unimpeded exposure to the vessel environment (see FIG. 7 as described below). Importantly, the configuration of FIGs. 7a-7c allow the portions of the optical fiber 30 containing the pressure-sensing FBGs 74 to be exposed in all directions to the interior of the vessel, which allows a single sensor station 70 and/or 72 to provide a 360 degree sensory window of the vessel lumen. In order to prevent the temperature-sensing FBGs 75 from being exposed to the pressure of the blood within the vessel, a pressure-isolating protective casing 33 may be constructed around these FBGs 75. This is best seen in FIG. 6c, in which the distal fiber optic wire 30 and related elements are shown together as an assembly as they would appear without the coils 29 of the guidewire core 22. The protective casing 33 has relatively high thermal conductivity so as to allow the temperature sensing FBGs 75 to sense the same temperature as their adjacent pressure-sensing FBGs 74.

It is possible for the pressure-sensing FBGs 74 to encounter strain as the distal fiber optic wire 30 passes into and through the vessel. Unfortunately, strain on the optic wire 30 at this location 74 can interfere with the ability of the pressure-sensing FBGs 74 to accurately measure the pressure inside the vessel. In the embodiment shown in FIGs. 6a, 6b, 6c, a strain reducing structure such as a cage 31 has been attached to the pressure-isolating protective casing 33 in order to isolate the pressure-sensing FBGs 74 from the effects of strain. As shown most clearly in FIG 6c, the cage 31 can take the form of one or more supporting elements extending between the protective casing 33 on one side of the pressure-sensing FBG 74 across to reconnect to the fiber optic wire 30 at the other side of the pressure-sensing FBG 74. The strain reducing structure 31 effectively forms a cage around the pressure-sensing FBG 74 which reduces strain at this location 74 while allowing full communication between the portion of the distal fiber optic wire 30 containing the pressure-sensing FBG 74 and the blood in the vessel. In some embodiments, this cage 31 is rigidly constructed so as to prevent any significant bending at the FBG 74.

In various embodiments the strain reducing structure 31 is sufficiently rigid to minimize or eliminate the effects of strain on those regions of the guidewire assembly 12 corresponding to the location of the FBGs 74. The structures 31 are also sufficiently short in length so as to not interfere with the flexibility of the guidewire assembly 12 and its ability to be advanced through the tortuous confines of the vascular anatomy. Example configurations of the structures 31 include the cage shown and described in FIG. 6c; and may include other configurations such as a hypotube or other structure disposed about the optic fiber 30 in the region of the FBG 74, while having one or more openings to provide exposure to the vascular environment. A hypotube environment may be constructed that allows the fiber optic wire 30 to travel through a lumen passing through the center of the hypotube. A window can be inserted into the top of the hypotube that exposes the top surface of the fiber optic wire at the location of the pressure-sensing FBG 74. In this environment, the hypotube continues to support the bottom surface of the fiber optic wire in order to reduce the strain on the fiber optic wire at this location.

In the above-described embodiments, a separate temperature-sensing FBG 75 is a part of each sensing station 70 and 72. This means that each pressure-sensing FBG 74 has a dedicated temperature-sensing FBG 75 that can compensate for changing temperatures in a location immediately proximate to the pressure-sensing FBG 74. In other embodiments a single temperature-sensing FBG 75 may be located at any point along the guidewire assembly 12, such as for example between sensor stations 70 and 72 and/or proximally adjacent and/or distally adjacent to either. In these embodiments, the single temperature-sensing FBG 75 is used to compensate for temperature variations for two or more pressure-sensing FBGs 74. Since the temperature-sensing FBG 75 need not be located near any particular pressure-sensing FBG 74, this construction may simplify the construction process of isolating the temperature-sensing FBG 75 from environmental pressure. In addition, using a single temperature-sensing FBG 75 will simplify construction of the distal fiber optic wire 30 by limiting the number of FBGs that must be created. Use of the guidewire assembly 12 is also simplified by limiting the number of different light wavelengths that must be emitted by the light source 40 and detected by the light wavelength detector 60. The disadvantage of using a single temperature-sensing FBG 75 is that the system 10 will not be able to accurately account for temperature variations experienced at different pressure-sensing FBGs 74.

In the various embodiments shown and described above the distal fiber optic wire 30 contains at least two sensor stations 70 and 72. As is shown in FIG. 7, in order to conduct simultaneous FFR the distal sensor station 70 is positioned distal or upstream of the affected region 102 and the proximal sensor station 72 is positioned proximal or downstream of the affected region 102. Light transmitted through the distal optical fiber 30 interacts with the FBGs 74 of each sensor station to provide a measurable pressure reading on each side of the affected region 102 simultaneously. Note: the phrase "simultaneous FFR" and the word "simultaneously" are used in this context to differentiate the system 10 of the present disclosure from conventional FFR systems and techniques. Known systems measure pressure (or other vessel characteristics) on each side of the affected regions at distinctly different times as necessitated by the need to reposition the guidewire and sensor mounted thereon. Embodiments of the present invention using two sensor stations 70 and 72, positioned in the manner shown in FIG. 2, to provide two sensed pressure values at essentially the speed of the light through the fiber optic wire 30. While there is a distance separating the two sensors 70 and 72, since the pressure sensors are in effect functioning at the speed of light, the difference in time between measurements is for all practical purposes indistinguishable, and are thus: simultaneous.

In a simultaneous FFR procedure, the pressure values provided from two FBGs (from sensor stations 70 and 72 in FIG. 7) are compared, and the resulting pressure difference is interpreted by programing of the system 10 and/or the physician 100 (see FIG. 1) to determine if the difference in pressure is indicative of a blockage (or other issue) requiring further therapeutic treatment. Thus, transmission, reflection, and analysis of light (peak shift) passed to and received from the sensor stations 70, and 72 provides simultaneous pressure readings from each station. The physician 100 (or the system 10) may calculate the pressure difference across the affected region 102 and subsequently determine if the difference is indicative of unacceptable flow restriction; and if so, and make a therapeutic decision to further treat the affected region such as by balloon angioplasty (POBA), stenting, drug delivery, or any combination thereof.

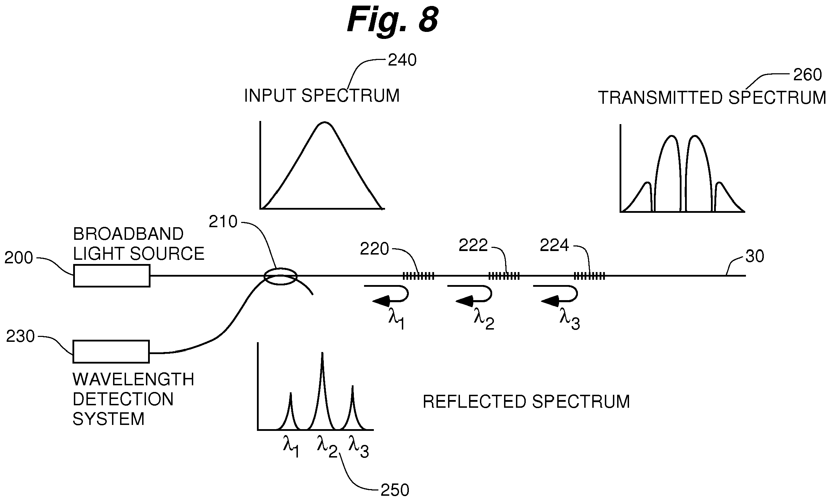

As mentioned above, sensor stations 70 and 72 each may include a pressure-sensing fiber Bragg grating (FBG) 74 and a temperature-sensing fiber Bragg grating 75. An FBG is a periodic modulation of the refractive index along a fiber optic core. The periodicity results in reflection of light waves that match the periodic spacing of the FBG wavelength while other wavelengths are transmitted unperturbed. The wavelength that is reflected by the FBG is determined by "effective refractive index" of the grating in the fiber core and the period of the grating. More particularly, an FBG in a standard, single mode fiber optic wire will reflect light waves of a wavelength centered around a single wavelength as determined by the effective refractive index and the period of the grating. By altering these elements, it is possible to configure a distal fiber optic wire 30 to contain multiple pressure-sensing FBGs 74 that each reflect light around a different wavelength. This is shown in FIG. 8, in which a broadband light source 200 is transmitted past a circulator 210 into the distal fiber optic wire 30. At each of three pressure-sensing FBGs 220, 222, 224, a different wavelength of light is reflected (namely λ₁, λ₂, and λ₃, respectively). Light around these wavelengths is reflected by the FBGs 220, 222, 224 and returns via the circulator 210 into the light or wavelength detection system 230. This system 230 analyzes the light it receives to identify the intensity of received light at various wavelengths. Using such a system 230, it is possible to analyze the wavelengths of light reflected from the FBGs 220, 222, 224. If a broadband light source is used, the chart of light intensity vs. wavelength of the input light is shown in chart 240 in FIG. 8. If each FBG 220, 222, 224 reflects light around a different wavelength, the chart of reflected light would show three peaks at λ₁, λ₂, and λ₃, as shown in chart 250. The light that passes through all three FBGs 220, 222, 224 is shown in chart 250.

It should be noted that in some embodiments distal and proximal fiber optic wire sections 30 and 32 are both single mode optical fibers. In some embodiments one or both fiber sections 30 and 32 (or portions thereof) may be configured as multi-mode fibers.

Various environmental conditions, such as temperature, pressure, and strain can alter the refractive index and grating period of the FBGs 74, 75 due to the photoelastic and thermooptical effects, which results in a small wavelength shift of the reflective peaks shown in chart 250. This shift can be detected, analyzed, and displayed as a value allowing the pressure-sensing FBG 74 to be used as a sensor. Unfortunately, the shift in reflected wavelength at a pressure-sensing FPG 74 is more sensitive to a change in temperature than it is to a change in pressure. This means that, unless there is a method to control for changes in temperature, it is extremely difficult to detect pressure changes by analyzing the wavelength of reflected light at the pressure-sensing FBGs 74. In the above described embodiments, a temperature-sensing FBG 75 is located proximal to each pressure-sensing FBG 74. The two FBGs 74, 75 are designed to reflect different wavelengths, and therefore are able to be separately analyzed by the light detection system 230. Because the temperature at a particular location will impact the temperature-sensing FBG 75 in the same manner as the pressure-sensing FBG 74, the wavelength shifts due to temperature will be practically identical at the two FBGs 74, 75. As a result, the movement of the wavelength peak in chart 250 of the temperature sensing FBG 75 can be used to identify the extent to which the wavelength peak of the pressure-sensing FBG 74 also moved as a result of temperature changes. In effect, wavelength shifts in the signal received from the temperature-sensing FBG 75 are used to cancel out the impact of temperature changes on the pressure-sensing FBG 74. Any movement in the wavelength peak of the pressure-sensing FBG 74 beyond that expected as a result of any temperature changes will be indicative of pressure from the blood contacting the distal fiber optic wire 30 at the location of the pressure-sensing FBG 74.

The pressure-related movement of the wavelength peaks due to pressure detected at FBG 74 can be converted into a pressure value being detected inside the vessel. In the preferred embodiment, the relationship between movement of wavelength peaks and pressure values are calibrated experimentally. While it is expected that the wavelength shift will be approximately linearly related to pressure, experimental calibration will allow for more complex relationships to be established for conversion between wavelength shift and pressure. In one embodiment, the light wavelength detector 60 includes light sensors that are sensitive to particular wavelengths of light as well as a processor that detects the wavelengths of the received light, analyzes the detected wavelengths, compensates for temperature-associated wavelength shifts detected in the temperature-sensing FBG 75, and applies the experimentally-determined conversion formula or table to convert the pressure-associated wavelength shift detected in the pressure-sensing FBG 74 into a pressure value. This processor could also compare pressure values from two sensor stations 70, 72 to determine a pressure difference at these two stations 70, 72, and determine whether this pressure difference is indicative of a need for further medical treatment. The processor and the sensor may be manufactured together into a single chip or component, or may be manufactured as separate chips and/or components that interact via known techniques for data communications.

The above analysis ignores any impact of strain on the wavelength of reflected light at the pressure-sensing FBG 74. As explained above, strain on the fiber optic wire 30 changes the refractive index and the grating period of the FBG in the fiber optic wire 30, and therefore can alter the wavelength of the reflected light at the pressure-sensing FBG 74. This means that strain caused by bending, stretching, or contracting the fiber optic wire 30 can impact the validity of the pressure measured at the pressure-sensing FBG 74. Such strain is likely to be present in any embodiment where the fiber optic wire is attached to a medical guidewire that has be advanced by a physician through a patient's vascular system to affected region of a vessel.

The above-described embodiments are designed to limit the strain on the fiber optic wire 30 to reduce the impact of strain on the resulting pressure measurement. In the embodiments shown in FIGs. 3a-3c and in FIGs. 4a-4c, the fiber optic wire 30 rests in a groove or channel 24 of the guidewire shaft 22. As shown, the fiber optic wire 30 is supported on three (out of four) sides, with only the top of the fiber optic wire 30 exposed. In this way, the groove or channel 24 helps support the fiber optic wire 30 and limits the strain on the fiber optic wire 30 as the guidewire shaft 22 passes through the patient's vascular system. Similarly, in the embodiment shown in FIGs. 5a and 5b, the fiber optic wire 30 is supported on all sides by the lumen 28 passing through the guidewire core 22. While the fiber optic wire 30 is exposed to the vessel through opening 26 at the location of the pressure-sensing FBG 74, the lumen 26 supports the guidewire on both sides of the pressure-sensing FBG 74. Furthermore, the opening 26 does not divide the guidewire shaft 22 into separately moveable elements, which might allow significant bending and strain on the fiber optic wire 30 at this location. Rather, as shown in FIG. 5a, the opening 26 merely bisects an integral portion of the shaft 22, allowing the single shaft element to support the fiber optic wire 30 on both sides of the pressure-sensing FBG 74. Finally, in the embodiment shown in FIGs. 6a-6c, the cage or strain reducing structure 31 is designed to hold the distal fiber optic wire 30 on both sides of the pressure-sensing FBG 74. The supporting elements that extend between the protective casing 33 on one side of the pressure-sensing FBG 74 across to reconnect to the fiber optic wire 30 at the other side of the pressure-sensing FBG 74 further helps to ensure that there is not significant bending and strain on the fiber optic wire 30 at this location.

As discussed above, one aspect of the present disclosure is the use of two sensors 70 and 72 to conduct a simultaneous FFR diagnostic procedure. It should also be noted, that many benefits of the system 10 apply equally to an embodiment having only a single sensor 70. Providing a guidewire assembly 12 with a single sensor 70 which can accurately detect a pressure value without interference from other vessel characteristics such as temperature and strain; and without the need of additional structures such as surrounding membranes is also an inventive aspect of the present system 10.

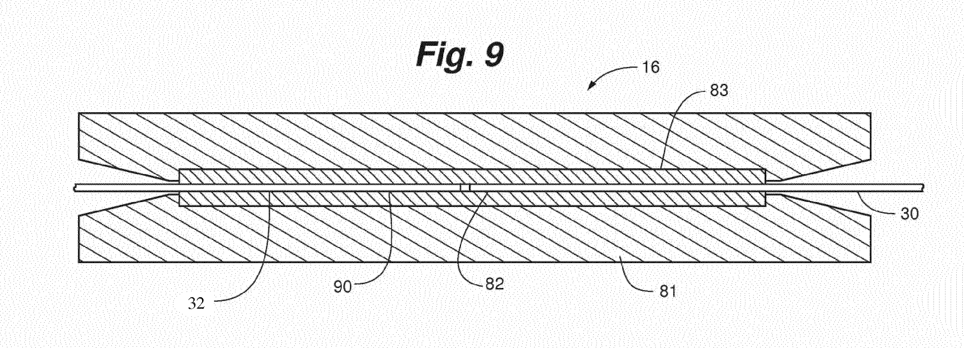

Returning to the system 10 as depicted in FIGs. 1-2, the guidewire assembly 12 is manipulated and repositioned as the distal end region of the assembly 12 is advanced through the vasculature to the site of a vessel lesion or other affected area 102. As the guidewire 20 is manipulated, the distal fiber optic wire 30 can be twisted and repositioned making it difficult or impossible to maintain its rotational orientation relative to the detection and analysis apparatus of the proximal assembly 14. To ensure proper transmission of light through the fiber optic line, the line includes a connector 16 which connects the distal fiber optic wire 30 to the proximal fiber optic wire 32. While the connector 16 may be configured merely as a connection between proximal fiber optic wire 32 and distal fiber optic wire 30 (such as is shown in FIG. 9); in some embodiments (such as are shown in FIGs. 1 and 2) the connector 16 comprises a mechanism for removably engaging the entire guidewire assembly 12 to and from the proximal assembly 14 so as to allow for greater ease of use during the advancement of the guidewire assembly 12 into position within the vasculature and to allow for use of the guidewire assembly 12 as a conventional guidewire.

In the embodiment depicted in FIGs. 1 and 2, the connector 16 is constructed of a male housing 80 and a female housing 86. The male housing 80 may be configured as the proximal-most end 36 of the guidewire assembly 12 and thus comprise the proximal end of the guidewire 20 and proximal end of the distal fiber optic wire 30. This male housing 80 is removably and rotatably engaged within a lumen 88 to the female housing 86. The female housing 86 contains the distal most end 90 of the proximal fiber optic wire 32. When the male housing 80 is engaged to the female housing 86, light is capable of being transmitted along the entire combined length of the optic fiber wires 30 and 32 while allowing the guidewire assembly 12 to be independently manipulated and rotated relative to the proximal assembly 14.

In at least one embodiment, the guidewire assembly 12 is disconnected from the connector 16 after the pressure analysis of the affected region 102 (see FIG. 7) is complete in order to allow the guidewire assembly 12 to act as a conventional guidewire for a subsequent angioplasty, stent delivery or other therapeutic procedure if needed. In at least one embodiment, such as is shown in FIG. 9 the connector 16 connects only the optic fibers 30 and 32, while the guidewire 20 remains disconnected or otherwise free to be manipulated by the physician 100 (see FIG. 1). In the embodiment shown in FIG. 9 the connector 16 comprises a single housing 81 which contains a micro-sleeve 83 into which the proximal end 82 of the distal fiber optic wire fiber 30 is inserted as well as the distal end 90 of the proximal fiber optic wire 32.

As shown in FIG. 2, the proximal optic wire 32 exits proximally from the connector 16 and extends to the proximal assembly 14. The proximal assembly 14 may be an assembly of individual components or may be combined into a single device. Some specific arrangements and example embodiments of the proximal assembly 14 are shown in FIG. 2. As will be understood by one of ordinary skill, the embodiments shown in FIG. 2 include components such as a light source 40, a circulator 52, and a light wavelength detector 60. In one embodiment, the light source 40 is a Superluminescent Light Emitting Diode (SLED). SLEDs emit a broad spectrum of light wavelengths, which ensure that light wavelengths appropriate for each FBG 74, 75 will enter into the distal optic fiber wire 30. Another embodiment uses a plurality of narrow linewidth lasers, each of which provide light for a single FBG 74, 75. A still further embodiment uses a scanning laser that sweeps across a plurality of wavelengths to ensure that the appropriate wavelengths for the FBGs 74, 75 are emitted into the distal optic fiber wire 30. As shown in FIG. 2, a fiber 32 exists to carry the light through the circulator 52 and into the connector 16. At the connector 16, light passes from the proximal optic wire 32 into the distal fiber optic wire 30. After the light is reflected from the FBGs 74, 75, the light passes back through the connector 16 and re-enters the proximal optic wire 32. The proximal optic wire 32 carries the light through the circulator 52 and into the detector 60.

The many features and advantages of the invention are apparent from the above description. Numerous modifications and variations will readily occur to those skilled in the art. Since such modifications are possible, the invention is not to be limited to the exact construction and operation illustrated and described. Rather, the present invention should be limited only by the following claims.

## Claims

1. A pressure sensing guidewire, comprising:
a) a coiled single filar or multi-filar guidewire (20) forming a lumen, wherein the coil of the guidewire comprises:
i) a first coiled frequency providing first openings of a first width between adjacent winds of the coiled single filar or the multi-filars; and
ii) at least one second coiled frequency that is less than the first coiled frequency, the second coiled frequency providing at least one second opening of a second width, greater than the first width, between adjacent winds of the coiled single filar or the multi-filars; and
b) a fiber optic wire (30) extending through the lumen of the coiled guidewire (20), the fiber optic wire (30) comprising:
a) a fiber core providing an effective refractive index to light having a specific wavelength;
b) a first pressure-sensing fiber Bragg grating, FBG, (74) at a first location on the fiber core of the fiber optic wire, wherein the first pressure-sensing FBG is laterally aligned with the at least one second opening of the second width of the coiled single filar or the multi-filars so that the first pressure-sensing FBG (74) is configured for 360° of direct exposure to environmental pressures adjacent thereto.

2. The pressure sensing guidewire of claim 1, wherein the first pressure-sensing FBG is restrained by a strain-resistant cage located within the lumen in the coiled guidewire, the strain-reducing cage encasing the fiber core of the fiber optic wire but having a cage opening so that the first pressure-sensing FBG is directly exposable to environmental pressure adjacent to the first location.

3. The pressure sensing guidewire of claim 2, wherein the first pressure-sensing FBG (74) and a temperature-sensing FBG (75) provide a periodic modulation of a refractive index along the fiber core resulting in reflection of light waves that match a periodic spacing of the first pressure-sensing FBG (74) and the temperature-sensing FBG (75) wavelengths, while other wavelengths are transmitted unperturbed.

4. The pressure sensing guidewire of claim 1, wherein a second pressure-sensing FBG resides at a second location on the fiber core of the fiber optic wire, the second pressure-sensing FBG being configured for direct exposure to environmental pressure adjacent thereto.

5. The pressure sensing guidewire of claim 1, wherein a temperature-sensing FBG resides at a third location on the fiber core of the fiber optic wire.

6. The pressure sensing guidewire of claim 5, wherein a pressure-isolating protective casing isolates the temperature-sensing FBG from direct exposure to environmental pressure adjacent thereto.

7. The pressure sensing guidewire of claim 1, wherein the fiber optic wire (30) is a single-mode fiber optic wire.

8. The pressure sensing guidewire of claim 1, wherein the first pressure-sensing FBG (74) of the first sensor station (70) on the fiber optic wire (30) is constrained in a strain reducing cage (31) in a lumen (28) of the guidewire (20).

9. The pressure sensing guidewire of claim 5, incorporated into a pressure sensing system (10), the pressure sensing system comprising:
a) a light source (40, 200);
b) a light wavelength detector (60);
c) a connector (16) positioned between a proximal assembly (14) comprising the light source (40) and the light wavelength detector (60), and a distal assembly (12) comprising the guidewire (20) supporting the fiber optic wire (30) as a distal fiber optic wire, wherein the connector (16) comprises:
i) a proximal housing (86) containing a proximal fiber optic wire (32); and
ii) a distal housing (80) that is removably mateable with the proximal housing (86), wherein a proximal end (82) of the distal fiber optic wire (30) supported by the guidewire (20) is contained in the distal housing (80), and
iii) wherein, when the distal housing (80) is mated with the proximal housing (86), the proximal end (82) of the distal fiber optic wire (30) contained in the distal housing (80) is in optical communication with a distal end (90) of the proximal fiber optic wire (32) contained in the proximal housing (86), and
d) wherein the first pressure-sensing FBG (74) and the first temperature-sensing FBG (75) are configured to reflect light in the distal fiber optic wire (30) to the proximal fiber optic wire (32) and then to the light wavelength detector (60), and
e) wherein the light wavelength detector (60) is configured to detect the reflected light and, after compensating for temperature based on reflected light from the temperature-sensing FBG (74), determine a pressure value at the first pressure-sensing FBG (74) of the distal fiber optic wire (30) after compensating for temperature based on the reflected light from the first temperature-sensing FBG (74).

10. The pressure sensing system of claim 9, wherein the connector (16) is configured to releasably and rotatably connect the distal fiber optic wire (30) to the proximal fiber optic wire (32).

11. The pressure sensing system of claim 9, wherein the proximal housing (86) is a female housing (86) and the distal housing (80) is a male housing (80), or wherein the proximal housing is a male housing and the distal housing (80) is a female housing.

12. The pressure sensing system of claim 9, wherein the proximal assembly (14) comprising the light source and the light wavelength detector further comprises a proximal single-mode fiber optic wire; and
wherein the distal fiber optic wire supported by the guidewire as the distal assembly is a single-mode fiber optic wire.

## Patentansprüche

1. Druckerfassungsführungsdraht, umfassend:
(a) einen Spulenmonofilar- oder Multifilar-Führungsdraht (20), der ein Lumen bildet, wobei die Spule des Führungsdrahts umfasst:
(i) eine erste Spulenfrequenz, die erste Öffnungen mit einer ersten Breite zwischen angrenzenden Windungen des Spulenmonofilars oder der Multifilare bereitstellt; und
(ii) mindestens eine zweite Spulenfrequenz, die geringer als die erste Spulenfrequenz ist, wobei die zweite Spulenfrequenz mindestens eine zweite Öffnung mit einer zweiten Breite, die größer als die erste Breite ist, zwischen angrenzenden Windungen des Spulenmonofilars oder der Multifilare bereitstellt; und
(b) einen Glasfaserdraht (30), der sich durch das Lumen des Spulenführungsdrahts (20) erstreckt, wobei der Glasfaserdraht (30) umfasst:
(a) einen Faserkern, der einen wirksamen Brechungsindex für Licht mit einer spezifischen Wellenlänge bereitstellt;
(b) ein erstes Druckerfassungs-Faser-Bragg-Gitter, FBG, (74) an einer ersten Stelle auf dem Faserkern des Glasfaserdrahts, wobei das erste Druckerfassungs-FBG lateral auf die mindestens eine zweite Öffnung mit der zweiten Breite des Spulenmonofilars oder der Multifilare ausgerichtet ist, so dass das erste Druckerfassungs-FBG (74) für 360° einer direkten Aussetzung gegenüber daran angrenzenden Umgebungsdrücken konfiguriert ist.

2. Druckerfassungsführungsdraht nach Anspruch 1, wobei das erste Druckerfassungs-FBG durch einen belastungsbeständigen Käfig zurückgehalten wird, der innerhalb des Lumens in dem Spulenführungsdraht angeordnet ist, wobei der belastungsbeständige Käfig den Faserkern des Glasfaserdrahts umschließt, jedoch eine Käfigöffnung aufweist, so dass das erste Druckerfassungs-FBG direkt gegenüber Umweltdruck, der an die erste Stelle angrenzt, aussetzbar ist.

3. Druckerfassungsführungsdraht nach Anspruch 2, wobei das erste Druckerfassungs-FBG (74) und ein Temperaturerfassungs-FBG (75) eine periodische Modulation eines Brechungsindexes entlang dem Faserkern bereitstellen, die zu einer Reflexion von Lichtwellen resultiert, die einem periodischen Abstand der Wellenlängen des ersten Druckerfassungs-FBG (74) und des Temperaturerfassungs-FBG (75) entsprechen, während andere Wellenlängen ungestört übertragen werden.

4. Druckerfassungsführungsdraht nach Anspruch 1, wobei ein zweites Druckerfassungs-FBG sich an einer zweiten Stelle auf dem Faserkern des Glasfaserdrahts befindet, wobei das Druckerfassungs-FBG für eine direkte Aussetzung gegenüber daran angrenzendem Umgebungsdruck konfiguriert ist.

5. Druckerfassungsführungsdraht nach Anspruch 1, wobei ein Temperaturerfassungs-FBG sich an einer dritten Stelle auf dem Faserkern des Glasfaserdrahts befindet.

6. Druckerfassungsführungsdraht nach Anspruch 5, wobei eine druckisolierende Schutzumhüllung das Temperaturerfassungs-FBG von einer direkten Aussetzung gegenüber daran angrenzendem Umgebungsdruck isoliert.

7. Druckerfassungsführungsdraht nach Anspruch 1, wobei der Glasfaserdraht (30) ein Monomode-Glasfaserdraht ist.

8. Druckerfassungsführungsdraht nach Anspruch 1, wobei das erste Druckerfassungs-FBG (74) der ersten Sensorstation (70) auf dem Glasfaserdraht (30) in einem belastungsreduzierenden Käfig (31) in einem Lumen (28) des Führungsdrahts (20) beschränkt wird.

9. Druckerfassungsführungsdraht nach Anspruch 5, der in ein Druckerfassungssystem (10) integriert ist, wobei das Druckerfassungssystem umfasst:
(a) eine Lichtquelle (40, 200);
(b) einen Lichtwellenlängendetektor (60);
(c) einen Verbinder (16), der zwischen einer proximalen Anordnung (14), die die Lichtquelle (40) und den Lichtwellenlängendetektor (60) umfasst, und einer distalen Anordnung (12), die den Führungsdraht (20) umfasst, der den Glasfaserdraht (30) als einen distalen Glasfaserdraht trägt, positioniert ist, wobei der Verbinder (16) umfasst:
(i) ein proximales Gehäuse (86), das einen proximalen Glasfaserdraht (32) enthält; und
(ii) ein distales Gehäuse (80), das abnehmbar mit dem proximalen Gehäuse (86) zusammengefügt werden kann, wobei ein proximales Ende (82) des distalen Glasfaserdrahts (30), der von dem Führungsdraht (20) getragen wird, in dem distalen Gehäuse (80) enthalten ist, und
(iii) wobei, wenn das distale Gehäuse (80) mit dem proximalen Gehäuse (86) zusammengefügt ist, das proximale Ende (82) des distalen Glasfaserdrahts (30), der in dem distalen Gehäuse (80) enthalten ist, in optischer Kommunikation mit einem distalen Ende (90) des proximalen Glasfaserdrahts (32), der in dem proximalen Gehäuse (86) enthalten ist, ist, und
(d) wobei das erste Druckerfassungs-FBG (74) und das erste Temperaturerfassungs-FBG (75) dazu konfiguriert sind, Licht in dem distalen Glasfaserdraht (30) zu dem proximalen Glasfaserdraht (32) und dann zu dem Lichtwellenlängendetektor (60) zu reflektieren, und
(e) wobei der Lichtwellenlängendetektor (60) dazu konfiguriert ist, das reflektierte Licht zu erfassen und nach Kompensieren in Bezug auf die Temperatur auf der Basis von reflektiertem Licht von dem Temperaturerfassungs-FBG (74) einen Druckwert an dem ersten Druckerfassungs-FBG (74) des distalen Glasfaserdrahts (30) nach Kompensieren in Bezug auf die Temperatur auf der Basis von reflektiertem Licht von dem ersten Temperaturerfassungs-FBG (74) zu bestimmen.

10. Druckerfassungssystem nach Anspruch 9, wobei der Verbinder (16) dazu konfiguriert ist, den distalen Glasfaserdraht (30) lösbar und drehbar mit dem proximalen Glasfaserdraht (32) zu verbinden.

11. Druckerfassungssystem nach Anspruch 9, wobei das proximale Gehäuse (86) ein Buchsengehäuse (86) ist und das distale Gehäuse (80) ein Steckergehäuse (80) ist oder wobei das proximale Gehäuse ein Steckergehäuse ist und das distale Gehäuse (80) ein Buchsengehäuse ist.

12. Druckerfassungssystem nach Anspruch 9, wobei die proximale Anordnung (14), die die Lichtquelle und den Lichtwellenlängendetektor umfasst, weiterhin einen proximalen Monomode-Glasfaserdraht umfasst und
wobei der distale Glasfaserdraht, der von dem Führungsdraht als die distale Anordnung getragen wird, ein Monomode-Glasfaserdraht ist.

## Revendications

1. Fil-guide de détection de pression, comprenant :
(a) un fil-guide unifilaire ou multifilaire enroulé (20) formant une lumière, dans lequel l'enroulement du fil-guide comprend :
(i) une première fréquence enroulée fournissant des premières ouvertures d'une première largeur entre des tours adjacents du fil unique ou des fils multiples enroulés ; et
(ii) au moins une deuxième fréquence enroulée qui est inférieure à la première fréquence enroulée, la deuxième fréquence enroulée fournissant au moins une deuxième ouverture d'une deuxième largeur, supérieure à la première largeur, entre des tours adjacents du fil unique ou des fils multiples enroulés ; et
(b) un fil de fibre optique (30) s'étendant à travers la lumière du fil-guide enroulé (20), le fil de fibre optique (30) comprenant :
(a) un cœur de fibre fournissant un indice de réfraction efficace à une lumière ayant une longueur d'onde spécifique ;
(b) un premier réseau de Bragg de fibre de détection de pression, RBF, (74) au niveau d'un premier emplacement sur le cœur de fibre du fil de fibre optique, dans lequel le premier RBF de détection de pression est aligné de manière latérale avec l'au moins une deuxième ouverture de la deuxième largeur du fil unique ou des fils multiples enroulés de sorte que le premier RBF de détection de pression (74) est configuré pour 360° d'exposition directe aux pressions environnementale adjacentes à celui-ci.

2. Fil-guide de détection de pression selon la revendication 1, dans lequel le premier RBF de détection de pression est retenu par une cage résistante à la déformation située au sein de la lumière dans le fil-guide enroulé, la cage de réduction de la déformation enveloppant le cœur de fibre du fil de fibre optique mais ayant une ouverture de cage de sorte que le premier RBF de détection de pression peut être exposé directement à la pression environnementale adjacente au premier emplacement.

3. Fil-guide de détection de pression selon la revendication 2, dans lequel le premier RBF de détection de pression (74) et un RBF de détection de température (75) fournissent une modulation périodique d'un indice de réfraction le long du cœur de fibre entraînant la réflexion d'ondes lumineuses qui correspondent à un espacement périodique des longueurs d'onde du premier RBF de détection de pression (74) et du RBF de détection de température (75), pendant que d'autres longueurs d'onde sont transmises sans être perturbées.

4. Fil-guide de détection de pression selon la revendication 1, dans lequel un deuxième RBF de détection de pression repose au niveau d'un deuxième emplacement sur le cœur de fibre du fil de fibre optique, le deuxième RBF de détection de pression étant configuré pour une exposition directe à la pression environnementale adjacente à celui-ci.

5. Fil-guide de détection de pression selon la revendication 1, dans lequel un RBF de détection de température repose au niveau d'un troisième emplacement sur le cœur de fibre du fil de fibre optique.

6. Fil-guide de détection de pression selon la revendication 5, dans lequel une enveloppe de protection d'isolation contre la pression isole le RBF de détection de température contre l'exposition directe à la pression environnementale adjacente à celui-ci.

7. Fil-guide de détection de pression selon la revendication 1, dans lequel le fil de fibre optique (30) est un fil de fibre optique monomode.

8. Fil-guide de détection de pression selon la revendication 1, dans lequel le premier RBF de détection de pression (74) de la première station de détecteur (70) sur le fil de fibre optique (30) est limité dans une cage de réduction de la déformation (31) dans une lumière (28) du fil-guide (20).

9. Fil-guide de détection de pression selon la revendication 5, incorporé dans un système de détection de pression (10), le système de détection de pression comprenant :
(a) une source de lumière (40, 200) ;
(b) un détecteur de longueurs d'ondes lumineuses (60) ;
(c) un connecteur (16) positionné entre un ensemble proximal (14) comprenant la source de lumière (40) et le détecteur de longueurs d'ondes lumineuses (60), et un ensemble distal (12) comprenant le fil-guide (20) supportant le fil de fibre optique (30) comme un fil de fibre optique distal, dans lequel le connecteur (16) comprend :
(i) un logement proximal (86) contenant un fil de fibre optique proximal (32) ; et
(ii) un logement distal (80) qui peut être accouplé de manière amovible avec le logement proximal (86), dans lequel une extrémité proximale (82) du fil de fibre optique distal (30) supporté par le fil-guide (20) est contenue dans le logement distal (80), et
(iii) dans lequel, quand le logement distal (80) est accouplé avec le logement proximal (86), l'extrémité proximale (82) du fil de fibre optique distal (30) contenue dans le logement distal (80) est en communication optique avec une extrémité distale (90) du fil de fibre optique proximal (32) contenue dans le logement proximal (86), et
(d) dans lequel le premier RBF de détection de pression (74) et le premier RBF de détection de température (75) sont configurés pour réfléchir la lumière dans le fil de fibre optique distal (30) au fil de fibre optique proximal (32) et puis au détecteur de longueur d'ondes lumineuses (60), et
(e) dans lequel le détecteur de longueurs d'ondes lumineuses (60) est configuré pour détecter la lumière réfléchie et, après la compensation de la température sur la base de la lumière réfléchie à partir du RBF de détection de température (74), déterminer une valeur de pression au niveau du premier RBF de détection de pression (74) du fil de fibre optique distal (30) après la compensation de la température sur la base de la lumière réfléchie à partir du premier RBF de détection de température (74).

10. Système de détection de pression selon la revendication 9, dans lequel le connecteur (16) est configuré pour connecter de manière libérable et rotative le fil de fibre optique distal (30) au fil de fibre optique proximal (32).

11. Système de détection de pression selon la revendication 9, dans lequel le logement proximal (86) est un logement femelle (86) et le logement distal (80) est un logement mâle (80), ou dans lequel le logement proximal est un logement mâle et le logement distal (80) est un logement femelle.

12. Système de détection de pression selon la revendication 9, dans lequel l'ensemble proximal (14) comprenant la source de lumière et le détecteur de longueurs d'ondes lumineuses comprend en outre un fil de fibre optique monomode proximal ; et
dans lequel le fil de fibre optique distal supporté par le fil-guide comme l'ensemble distal est un fil de fibre optique monomode.
